# EUROPÄISCHE PATENTSCHRIFT

(11) **EP 2 022 517 B1**
(45) Veröffentlichungstag und Bekanntmachung des Hinweises auf die Patenterteilung: **21.04.2010**
(21) Anmeldenummer: 08013711.0
(22) Anmeldetag: 31.07.2008
(51) Int. Cl.: A61M 1/36, A61M 1/34

(54) **Blutschlauchsystem für extrakorporale Anwendungen wie Dialysegeräte, einschließlich einer Expansionskammer mit einer kleinen Blut-Luft-Kontaktfläche.**
Blood tube system for extracorporeal applications such as dialysis devices, comprising an expansion chamber providing a small air-blood contact surface
Tube de prélèvement sanguin pour applications extracorporelles comme les appareils de dialyse, comprenant une chambre d'expansion ayant une surface de contact air-sang réduite.

(30) Priorität: 01.08.2007 DE 102007036125
(43) Veröffentlichungstag der Anmeldung: 11.02.2009
(73) Patentinhaber: Becker, Franz Ferdinand, 63110 Rodgau (DE); Ryzlewicz, Thomas, 83129 Höslwang (DE); Herbst, Reinhold H., 87700 Memmingen (DE)
(72) Erfinder: Becker, Franz Ferdinand, 63110 Rodgau (DE); Ryzlewicz, Thomas, 83129 Höslwang (DE); Herbst, Reinhold H., 87700 Memmingen (DE)
(74) Vertreter: Flosdorff, Jürgen

(56) Entgegenhaltungen:
- EP-A- 0 143 064
- EP-A- 1 787 666
- WO-A-2004/000391
- US-A- 5 252 213

## Beschreibung

Die Erfindung betrifft ein Blutschlauchsystem für extrakorporale Anwendungen wie Dialysegeräte.

Die Erfindung wird nachfolgend in Zusammenhang mit ihrer Anwendung auf ein Blutschlauchsystem für Dialysegeräte beispielhaft beschrieben.

Ein solches Blutschlauchsystem ist in dem Dokument EP 1 787 666 offenbart.

Bei einer Dialysebehandlung wird bisher eine beträchtliche Menge Heparin eingesetzt, wobei auch EPO in nennenswerter Menge verabreicht wird, um Thrombosen zu verhindern. Diese Stoffe können auf die Dauer die Gesundheit eines Patienten beeinträchtigen, und sie sind auch mit nicht unerheblichen Kosten verbunden.

Der vorliegenden Erfindung liegt die Aufgabe zugrunde, eine Lösung zu beschreiben, bei der die benötigte Heparin- und EPO-Menge reduziert werden kann.

Diese Aufgabe wird erfindungsgemäß durch die Merkmale des Patentanspruchs 1 gelöst.

Vorteilhafte Ausgestaltungen der Erfindung sind in den Unteransprüchen gekennzeichnet.

Die Erfindung sieht vor, dass das Blutschlauchsystem eine arterielle Expansionskammer hat, die einen unteren Bereich zur Aufnahme von Blut aufweist, in den ein Bluteinlaufkanal einmündet und von dem ein Blutauslaufkanal abgeht, und die einen oberen Bereich zur Aufnahme von Luft enthält, wobei von dem oberen Bereich ein mit einem Entlüftungsventil versehener Entlüftungskanal abgeht. Erfindungsgemäß hat die Expansionskammer einen mittigen im wesentlichen rohrförmigen Bereich mit erheblich verringerten Querschnittsabmessungen, wobei während einer Behandlung der Blutspiegel in der arteriellen Expansionskammer durch das Entlüftungsventil so eingestellt wird, dass der Blut-Luft-Kontakt in diesem mittigen eingeschnürten Bereich erfolgt. Dabei hat der mittige Bereich vorzugsweise die Form eines zylindrischen Rohres mit kreisförmigem oder ovalem Querschnitt und kann als "Steigrohr" bezeichnet werden. Der eingeschnürte Bereich kann aber beispielsweise in vertikaler Richtung leicht konisch verlaufen. Der Durchmesser dieses im wesentlichen rohrförmigen Bereichs kann etwa mit dem Durchmesser des Bluteinlaufkanals und des Blutauslaufkanals übereinstimmen, ohne dass die Erfindung hierauf beschränkt ist.

Das Blut fließt beim Füllvorgang durch einen Zufuhrstutzen, der vorzugsweise durch den Boden der Expansionskammer hindurch ins Innere des unteren Bereichs der Expansionskammer ragt, in die Expansionskammer hinein, wobei mit dem Entlüftungsventil der Blutspiegel so eingestellt wird, das er sich etwa in der Mitte des Steigrohres befindet. Während einer Dialyse bewegt sich der Blutspiegel, bedingt durch eine pulsierende Pumpe des Blutschlauchssystems, in dem Steigrohr auf und ab, wobei das Steigrohr (oder allgemein ausgerückt: der mittige eingeschnürte Bereich) eine solche Länge hat, dass der Blutspiegel im Bereich des Steigrohres verbleibt. Die genaue Einstellung erfolgt zu Beginn einer Behandlung durch Öffnen des Entlüftungsventils.

Die Länge des Steigrohres und dessen Durchmesser hängen von dem jeweiligen Blutschlauchsystem und Dialysegerät ab und können durch experimentelle Untersuchungen ermittelt werden. In einer bevorzugten Ausgestaltung beträgt die Querschnittsfläche des mittigen im wesentlichen rohrförmigen Bereichs etwa 1/7 der Querschnittsfläche des unteren Bereichs der Expansionskammer.

Durch die erfindungsgemäße Ausbildung der Expansionskammer entsteht eine kleine Blut-Luft-Kontaktfläche, wodurch die Thrombosierungsgefahr deutlich verringert wird.. Es hat sich herausgestellt, das beim Einsatz dieser Expansionskammer der Verbrauch an Heparin und EPO und anderen Substanzen pro Patient und Behandlung deutlich reduziert werden kann.

Der untere Bereich der Expansionskammer geht in gewölbter Form, vorzugsweise in Form eines Kugelabschnitts, in das Steigrohr über, wodurch das Blut im unteren Bereich der Expansionskammer verwirbelt wird. Dies wird dadurch verstärkt, dass der Blutzufluss bevorzugt in der Nähe des Randes des unteren Bereichs erfolgt.

In einer bevorzugten Ausführungsform ist vorgesehen, dass der mittige eingeschnürte Bereich der Expansionskammer durch einen ringförmigen Einsatz begrenzt ist, der in der Expansionskammer in geeigneter Höhe befestigt ist. Dieser ringförmige Einsatz kann ebenso wie die eigentliche Expansionskammer aus Kunststoff bestehen. Die Expansionskammer hat zweckmäßigerweise eine Zylinderform mit gerundeten Rändern am Übergang der oberen und unteren Wand in die Umfangswand. Die zylindrische Expansionskammer hat vorzugsweise einen ovale oder kreisförmige Querschnittsform.

Weiter wird mit Vorteil vorgeschlagen, das der ringförmige Einsatz an der Oberseite und an der Unterseite konkav geformte Wandflächen aufweist, in deren Mitte der steigrohrförmige Durchlasskanal liegt. Der ringförmige Einsatz ist zweckmäßigerweise ein Hohlkörper, der mit seiner Umfangswand an der Innenwand der Expansionskammer auf geeignete Weise befestigt ist, beispielsweise mit Hilfe eines Klebemittels oder durch Thermoschweißen etc.

Nach einem weiteren Vorschlag der Erfindung kann die Expansionskammer mit einem seitlichen Füllgriff versehen sein, der zweckmäßigerweise im unteren Bereich seitlich angeformt oder angesetzt ist. Dieser Füllgriff ermöglicht es, die arterielle Expansionskammer beim Füllen leicht zu neigen, wodurch der Füllvorgang erleichtert ist. Außerdem kann der Füllgriff dazu dienen, die Expansionskammer in einem dafür vorgesehenen Halter in einer definierten Position auszurichten. Auch dies ist eine Erleichterung für den Anwender.

Nach einem weiteren Vorschlag der Erfindung kann das Blutschlauchsystem auch eine venöse Expansionskammer, vorzugsweise aus Kunststoff, haben, die sich wie die arterielle Expansionskammer im Überdruckbereich befindet, wo der Eintritt von Luft aus der Umgebung ausgeschlossen werden kann. Die venöse Expansionskammer hat erfindungsgemäß einen zylindrischen oberen Bereich mit einem Kammervolumen, das etwa auf zwei Drittel einer herkömmlichen venösen Expansionskammer reduziert ist, und einen konischen unteren Bereich, von dem der Blutauslaufkanal abgeht. Über einen Blutzuflusskanal wird die venöse Expansionskammer vollständig mit Blut gefüllt, wobei über eine ebenfalls an der Oberseite der venösen Expansionskammer angeordnete Druckmessleitung der Druck gemessen wird. Diese Druckmessleitung hat eine Länge von mindestens 60 Millimeter, wodurch verhindert wird, dass Blut an den Druckkonektor gelangt. Außerdem hat die venöse Expansionskammer in der Oberseite einen Infusionszugang, durch den notwendige Infusionen zugeführt werden können. In dem unteren Kammertrichter kann das bei herkömmlichen Expansionskammern vorhandene thrombosierende Sieb entfallen, wodurch ebenfalls die während einer extrakorporalen Blutbehandlung erforderliche Heparinmenge beträchtlich reduziert werden kann.

Weitere Einzelheiten der Erfindung ergeben sich aus der nachfolgenden Beschreibung sowie anhand der Zeichnungen. Dabei zeigen:
Fig. 1 eine Ausführungsform einer erfindungsgemäßen arteriellen Expansionskammer;
Fig. 2 einen Schnitt A-A durch die arterielle Expansionskammer gemäß Fig. 1 und

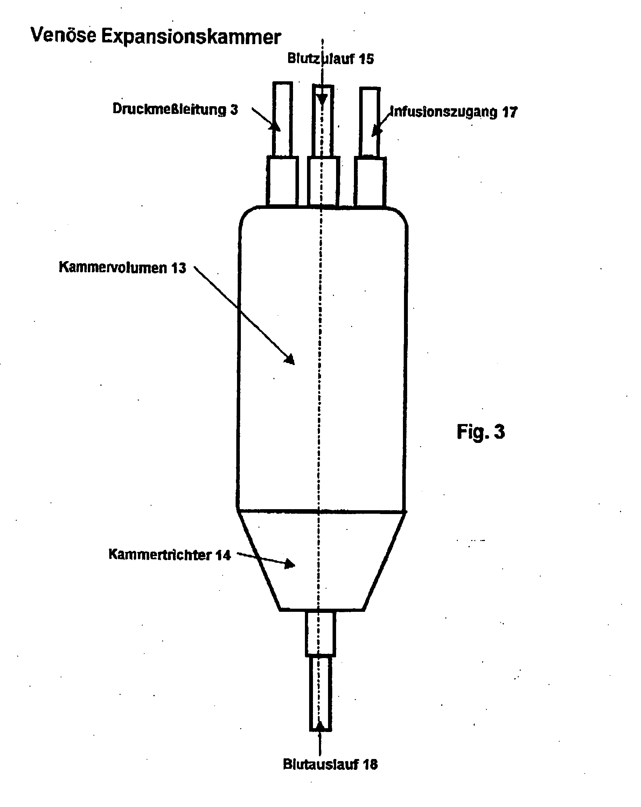

Fig. 3 eine Ausführungsform einer erfindungsgemäßen venösen Expansionskammer.

Die arterielle Expansionskammer hat eine Zylinderform mit ovalem Querschnitt, wobei in die arterielle Expansionskammer ein ringförmiger Einsatz 1 eingesetzt ist. Dieser ringförmige Einsatz hat oben und unten konkav gerundete Flächen 2, die in einen steigrohrartigen mittigen Kanal 3 einmünden.

Der steigrohrartige Kanal 3 verbindet einen unteren Bereich 4 der Expansionskammer, der mit Blut gefüllt wird, mit einem oberen Bereich 5, der mit Luft gefüllt ist. Vom oberen Bereich 5 geht ein Entlüftungskanal 6 ab, der mit einem Entlüffiungsventil 7 versehen ist und an der Oberseite durch eine abnehmbare Kappe 8 verschließbar ist.

Durch den Boden der Expansionskammer führt ein Bluteinlaufstutzen 9, der in den unteren Bereich 4 hineinragt und durch den Blut in die arterielle Expansionskammer eintritt, das durch einen Blutauslaufkanal 10 wieder abfließt. Der Blutauslaufkanal 10 ragt nicht ins Innere des unteren Bereichs 4 vor. Das Blut fließt beim Füllvorgang durch den mit einer Anschrägung versehen, längeren Zufuhrstutzen 9 in die Expansionskammer ein, wobei durch Öffnen des Entlüftungsventils 7 (nach Abnahme der Kappe 8) der Blutspiegel 11 innerhalb der Expansionskammer so eingestellt wird, das er etwa auf mittlerer Höhe des Steigrohres 3 liegt. Das Steigrohr 3 hat eine solche Länge, dass der Blutspiegel 11 während seiner pulsierenden Aufwärts- und Abwärtsbewegung stets im Bereich des Steigrohres 3 verbleibt. Dies hat zur Folge, dass die Kontaktfläche zwischen dem Blut und der in dem oberen Bereich 5 der Expansionskammer vorhandenen Luft minimiert ist.

An der Seite der Expansionskammer ist ein Füllgriff 12 angesetzt, der den Füllvorgang erleichtert und es ermöglicht, dass die Expansionskammer in definierter Lage in einen in der Zeichnung nicht dargestellten Halter eingesetzt werden kann.

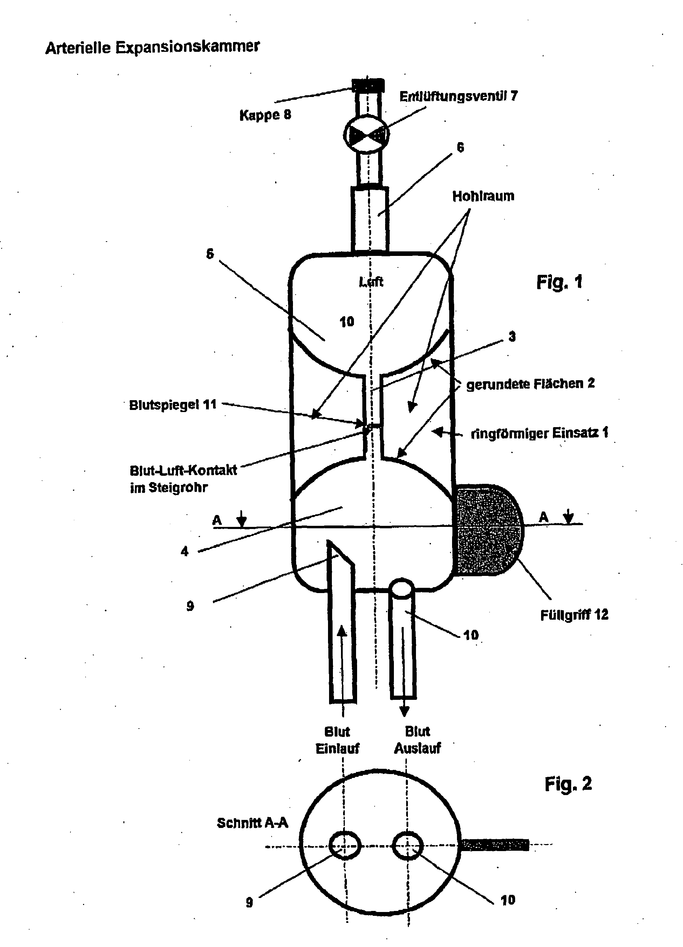

Fig. 2 zeigt eine venöse Expansionskammer, die eine obere zylindrische Kammer 13 mit einem gegenüber herkömmlichen venösen Expansionskammern reduzierten Volumen und einen unteren Kammertrichter 14 enthält. Durch einen Blutzulaufkanal 15 wird die venöse Expansionskammer vollständig mit Blut gefüllt, deren Druck durch eine obere Druckmessleitung 16 messbar ist.

Durch die obere Wand der venösen Expansionskammer führt außerdem ein Infusionszugang 17.

Vom Boden des Kammertrichters 14 geht ein Blutauslaufkanal 18 ab, der mit dem Blutzulaufkanal 15 fluchtet.

In dem Kammertrichter 14 kann das bisher üblicherweise vorhandene thrombosierende Sieb entfallen, wodurch die Heparinmenge, die während der Dialysebehandlung notwendig ist, reduzierbar ist.

Es wird betont, dass die Erfindung nicht auf die beschriebenen und dargestellten Ausführungsformen beschränkt ist. Vielmehr sind alle offenbarten Merkmale auf jede Weise einzeln miteinander kombinierbar.

## Patentansprüche

1. Blutschlauchsystem für extrakorporale Anwendungen wie Dialysegeräte, mit einer arteriellen Expansionskammer, die einen unteren Bereich zur Aufnahme von Blut aufweist, in den ein Bluteinlaufkanal einmündet und von dem ein Blutauslaufkanal abgeht, ferner mit einem oberen Bereich zur Aufnahme von Luft, wobei von dem oberen Bereich ein mit einem Entlüftungsventil (7) versehener Entlüftungskanal abgeht,
**dadurch gekennzeichnet,**
**dass** ein in vertikaler Richtung mittiger, im wesentlichen rohrförmiger Bereich (3) der Expansionskammer ausgebildet ist, der im Vergleich zu den anschließenden oberen und unteren Bereichen der Expansionskammer verringerte Querschnittsabmessungen hat, und
**dass** der Blutspiegel (11) durch das Entlüftungsventil (7) in der Höhe so einstellbar ist, dass der Blut-Luft-Kontakt während einer Dialyse in dem mittigen rohrförmigen Bereich (3) erfolgt.

2. Blutschlauchsystem nach Anspruch 1,
**dadurch gekennzeichnet,**
**dass** der mittige Bereich die Form eines zylindrischen Steigrohres (3) hat.

3. Blutschlauchsystem nach Anspruch 1 oder 2,
**dadurch gekennzeichnet,**
**dass** der mittige Bereich durch einen ringförmigen Einsatz (1) begrenzt ist, der in der Expansionskammer befestigt.

4. Blutschlauchsystem nach Anspruch 3,
**dadurch gekennzeichnet,**
**dass** der ringförmige Einsatz (1) an seiner Oberseite und an seiner Unterseite konkav geformte Wandflächen (2) aufweist.

5. Blutschlauchsystem nach einem der Ansprüche 1 - 4,
**dadurch gekennzeichnet,**
**dass** der Bluteinlaufkanal (9) mit einem Zufuhrstutzen von unten her durch den Boden der Expansionskammer hindurch ins Innere des unteren Bereichs (4) der Expansionskammer ragt.

6. Blutschlauchsystem nach Anspruch 5,
**dadurch gekennzeichnet,**
**dass** das Ende des Zufuhrstutzens (9) abgeschrägt ist.

7. Blutschlauchsystem nach einem der Ansprüche 1-6,
**dadurch gekennzeichnet,**
**dass** die Expansionskammer mit einem seitlichen Füllgriff (12) versehen ist.

8. Blutschlauchsystem nach Anspruch 1,
**dadurch gekennzeichnet,**
**dass** ferner eine venöse Expansionskammer vorgesehen ist, die einen zylindrischen oberen Bereich (13), in den ein Blutzulaufkanal (15) einmündet, und einen konischen unteren Bereich (14) aufweist, von dem ein Blutauslaufkanal (18) abgeht.

## Claims

1. A blood tube system for extracorporeal uses, such as dialysis devices, comprising an arterial expansion chamber including a lower region for receiving blood, in which a blood inlet channel terminates and from which a blood outlet channel exits, further an upper region for receiving air, a venting channel provided with a venting valve (7) extending from the upper region,
**characterized in**
**that** a vertically central, substantially tubular region (3) of the expansion chamber is formed, which in comparison with the adjoining upper and lower regions of the expansion chamber has reduced cross-sectional dimensions, and
**that** the blood level (11) is adjustable by way of the venting valve (7) in its height in such a manner that blood/air contact during dialysis takes place in the central tubular region (3).

2. The blood tube system according to claim 1,
**characterized in**
**that** the central region has the form of a cylindrical riser tube (3).

3. The blood tube system according to claim 1 or 2,
**characterized in**
**that** the central region is defined by an annular insert (11) which is fixed in the expansion chamber.

4. The blood tube system according to claim 3,
**characterized in**
**that** the annular insert (1) is provided at its upper side and at its lower side with concavely shaped wall surfaces (2).

5. The blood tube system according to any one of claims 1 to 4,
**characterized in**
**that** the blood inlet channel (9) projects with a supply nozzle from underneath through the bottom of the expansion chamber into the interior of the lower region (4) of the expansion chamber.

6. The blood tube system according to claim 5,
**characterized in**
**that** the end of the supply nozzle (9) is beveled.

7. The blood tube system according to any one of claims 1 to 6,
**characterized in**
**that** the expansion chamber is provided with a lateral filling handle (12).

8. The blood tube system according to claim 1,
**characterized in**
**that** a venous expansion chamber is further provided which comprises a cylindrical upper region (13), in which a blood inlet channel (15) terminates, and a conical lower region (14), from which the blood outlet channel (18) extends.

## Revendications

1. Système tubulaire de circulation du sang pour des applications extracorporelles, telles que des appareils de dialyse, comportant une chambre d'expansion artérielle, qui possède une zone inférieure destinée à recevoir le sang, dans laquelle débouche un conduit d'admission du sang et de laquelle part un conduit d'évacuation du sang, et possède une zone supérieure destinée à recevoir de l'air, sachant qu'un conduit de purge d'air, muni d'une vanne de purge d'air (7), part de la zone supérieure,
**caractérisé**
**en ce qu'**il est réalisé une zone (3) centrale dans la direction verticale, sensiblement tubulaire, dans la chambre d'expansion, laquelle zone a une section avec des dimensions inférieures à celles des zones supérieures et inférieures adjacentes de la chambre d'expansion, et
**en ce que** le niveau du sang (11) peut être réglé en hauteur par la vanne de purge d'air (7), de telle sorte que le contact sang-air pendant une dialyse se produit dans la zone (3) centrale tubulaire.

2. Système tubulaire de circulation du sang selon la revendication 1, **caractérisé en ce que** la zone centrale a la forme d'une colonne montante (3) cylindrique.

3. Système tubulaire de circulation du sang selon la revendication 1 ou 2, **caractérisé en ce que** la zone central est délimitée par un insert (1) annulaire, qui est fixé dans la chambre d'expansion.

4. Système tubulaire de circulation du sang selon la revendication 3, **caractérisé en ce que** l'insert (1) annulaire possède des surfaces de paroi (2) à forme concave sur sa face supérieure et sa face inférieure.

5. Système tubulaire de circulation du sang selon l'une quelconque des revendications 1 à 4, **caractérisé en ce que** le conduit d'admission du sang (9) s'engage avec une tubulure d'admission depuis le bas à travers le fond de la chambre d'expansion vers l'intérieur de la zone inférieure (4) de la chambre d'expansion.

6. Système tubulaire de circulation du sang selon la revendication 5, **caractérisé en ce que** l'extrémité de la tubulure d'admission (9) est chanfreinée.

7. Système tubulaire de circulation du sang selon l'une quelconque des revendications 1 à 6, **caractérisé en ce que** la chambre d'expansion est munie d'une manette de remplissage (12) latérale.

8. Système tubulaire de circulation du sang selon la revendication 1, **caractérisé en ce qu'**il est prévu, en outre, une chambre d'expansion veineuse, qui possède une zone supérieure (13) cylindrique, dans laquelle débouche un conduit d'admission du sang (15), et une zone inférieure (14) conique, de laquelle part un conduit d'évacuation du sang (18).
